Europäisches Patentamt

(19) European Patent Office

Office européen des brevets

(11) Publication number: **0 028 563**
**B1**

(12) **EUROPEAN PATENT SPECIFICATION**

(45) Date of publication of patent specification: **04.04.84**   (51) Int. Cl.³: **A 61 K 39/12**

(21) Application number: **80401527.9**

(22) Date of filing: **28.10.80**

(54) Stabilizer for liquid vaccines and liquid vaccine containing said stabilizer.

(30) Priority: **29.10.79 US 89068**
**05.02.80 US 118703**

(43) Date of publication of application:
**13.05.81 Bulletin 81/19**

(45) Publication of the grant of the patent:
**04.04.84 Bulletin 84/14**

(84) Designated Contracting States:
**AT BE CH DE FR GB IT LI LU NL SE**.

(56) References cited:
BE - A - 641 705
BE - A - 665 076
BE - A - 866 330
DE - A - 2 816 349
FR - A - 2 076 787
FR - A - 2 115 379
FR - A - 2 215 946
FR - A - 2 216 997
FR - A - 2 424 031
LU - A - 79 529
US - A - 4 147 772

(73) Proprietor: **MERCK & CO. INC.**
**126, East Lincoln Avenue P.O. Box 2000**
**Rahway New Jersey 07065 (US)**

(72) Inventor: **McAleer, William J.**
**717 Marietta Drive**
**Ambler Pennsylvania 19002 (US)**
Inventor: **Markus, Henry Z.**
**1517 Thornberry Road**
**Wyncote Pennsylvania 19095 (US)**

(74) Representative: **Corre, Jacques Denis Paul et al,**
**Cabinet Regimbeau 26, Avenue Kléber**
**F-75116 Paris (FR)**

(56) References cited:
**CHEMICAL ABSTRACTS, vol. 80, no. 8, February
25, 1974, abstract 41031f, page 269,
COLUMBUS, OHIO (US)**

Courier Press, Leamington Spa, England.

Stabilizer for liquid vaccines and liquid vaccine containing said stabilizer.

Background of the invention

The present invention relates to liquid viral vaccines. More particularly it relates to a stabilizer for liquid viral vaccines.

Due to the worldwide distribution of vaccines and the diversity of ambient temperatures, there has been a need to stabilize these preparations for transportation and use. Several stabilization methods have been used in the past.

a) Low temperatures (—10°C to —70°C)

The need for low temperature storage facilities which are not always available limits the practicality of this approach.

b) Lyophilization

Although lyophilization suffers the disadvantages of being an expensive procedure, lyophilized vaccines are reasonably stable and are stored at 4—8°C. until needed. During this storage period, however, the vaccines slowly deteriorates until after about 12—24 months it does not have sufficient titer to confer immunization. Furthermore, since the lyophilized vaccine must be reconstituted prior to use, the liquid reconstituted preparation loses potency while standing at room temperature. This can result in insufficient titer to confer immunity and results in failure of immunization program.

c) Stabilizers

These are chemical compounds added to the vaccine and are used in conjunction with either lower temperature storage or lyophilization methods. Chemical stabilizers, e.g., SPGA (a stabilizer described by Bovarnick et al., J. Bact. 59:509—522 (1950), the disclosure of which is incorporated herein by reference) and the like are described in the prior art. As described by Bovarnick et al. a liter of SPGA contains 0.218 M sucrose (74.62 g), 0.00376 M $KH_2PO_4$ (0.52 g), $K_2HPO_4$ 0.0071 M (1.25 g), potassium glutamate 0.0049 M (0.912 g) and 1% serum albumin (10 g). Various modifications of the foregoing amounts of ingredients of SPGA are known to those skilled in the art and sodium glutamate is frequently substituted for potassium glutamate, but the modified compositions are still designated as SPGA. For example, U.S.—A—3,783,098 refers to an SPGA stabilizer containing monosodium glutamate rather than monopotassium glutamate (col. 6, lines 5—11). A known SPGA stabilizer contains per liter of sterile distilled water, 74.62 g sucrose, 0.45 g $KH_2PO_4$, 1.35 g $K_2HPO_4$, 0.956 g monosodium L-glutamate, and 40 ml of a 25% solution of albuminsol (human albumin). In general an SPGA stabilizer contains from about 2 to 10% of sugar, e.g. sucrose; from 0.05 to 0.3% of a mono- or dibasic alkali metal phosphate salt or mixture thereof, e.g. $KH_2PO_4$, $K_2HPO_4$, $NaH_2PO_4$, or $Na_2HPO_4$, from 0.05 to 0.2% of a glutamic acid alkali metal salt, e.g. sodium or potassium glutamate; and from 0.5% to 2% serum albumin, e.g. bovine serum albumin or human albumin. Various substitutions of ingredients in the formulation of SPGA stabilizer can be made. For example, a starch hydrolysate, e.g. glucose or dextran may be substituted wholly or partly for sucrose as disclosed in U.S.—A—3,783,098 (col. 3, lines 59—61) and casein or PVP may be substituted wholly or partly for albumin as described respectively, in U.S.—A—3,783,098 (col. 3, line 8) and U.S.—A—3,915,794. However, none of the prior art stabilizers also described in Chemical Abstracts 80 (1974), 41031f, US—A—4,147,772, BE—A—665 076, and US—A—2,115,379, imparts the desired enhanced sustained level of stability.

Objects of the invention

It is, accordingly, an object of the present invention to provide an improved chemical stabilizer for liquid viral vaccines. Another object is to provide a method for stabilizing liquid viral vaccines. A further object is to provide liquid viral vaccines having prolonged storage stability with diminished reduction in titer. These and other objects of the present invention will be apparent from the following description.

The present invention is directed to a liquid vaccine comprising an inactivated or attenuated virus and a stabilizer consisting essentially of on a parts by weight basis from 1.5 to 2.1 parts partially hydrolyzed gelatin having a molecular weight of 3,000 from 7.0 to 13.0 parts of a monosaccharide, disaccharide or the polyhydric alcohol sorbitol from 0.4 to 0.6 part of an *in vitro* cell culture medium, and an amount of a physiologically acceptable acidic buffer effective to maintain the pH to from 6.0 to 6.5, characterized by the fact it also comprises from 0.35 to 0.7 part L-glutamic acid, from 0.75 to 1.3 parts L-arginine.

The liquid vaccine may be obtained by thawing a frozen vaccine or by reconstituting a lyophilized vaccine. Examples of virus are measles, mumps, rubella, varicella, polio, or hepatitis or a combination of any two or more of such viruses. Hydrolyzed gelatin is employed to provide a soluble, nongelling proteinaceous matrix with little or no pyrogenicity or antigenicity.

By partially hydrolyzed gelatin is meant gelatin which has been subjected to partial hydrolysis to yield a partially hydrolyzed gelatin having a molecular weight of about 3,000. This gelatin hydrolysis product has approximately the same amino acid composition as gelatin. Unlike gelatin which forms gels but is insoluble in cold water, hydrolyzed gelatin does not gel but is soluble in cold water, and other common liquids such as milk and orange juice. Aqueous solutions containing up to 10% hydrolyzed gelatin do not increase appreciably in viscosity. Above 10% concentration, viscosity increases slowly. At 50% concentration, solutions are quite viscous. The typical amino-acid composition of hydrolyzed gelatin follows:

| | |
|---|---|
| Alanine | 8.5% |
| Arginine | 7.9% |
| Aspartic Acid | 5.7% |
| Cystine | 0.08% |
| Glutamic Acid | 9.5% |
| Glycine | 22.8% |
| Histidine | 0.77% |
| Hydroxy Proline | 13—14% |
| Isoleucine | 1.3% |
| Leucine | 2.9% |
| Lysine | 4.2% |
| Methionine | 0.78% |
| Phenyl Alanine | 2.0% |
| Proline | 13.8% |
| Serine | 3.3% |
| Threonine | 1.9% |
| Tyrosine | 0.40% |
| Valine | 2.4% |

Partially hydrolyzed gelatin may be obtained by enzymatic hydrolysis of gelatin by means of a proteolytic enzyme, such as, for example, papain, chymopapain and bromelin, although other known hydrolysis means may be employed, e.g. acid hydrolysis. A suitable hydrolyzed gelatin is obtainable from Wilson and Co., Inc., Calumet City, Illinois under the trade name SOL-U-PRO.

The stabilizer also includes a monosaccharide, e.g. the polyhydric alcohol sorbitol, or a disaccharide, e.g., sucrose, lactose, or maltose. Sucrose is preferred.

The L-glutamic acid may be used as such or in the form of its sodium salt, monosodium glutamate.

The L-arginine may be used as such or in the form of its hydrochloride salt.

The acidic buffer may be any physiologically acceptable buffer which will maintain the desired pH of from 6 to 6.5, for example, phosphate buffer, acetate buffer or citrate buffer. Phosphate buffer is preferred. The stabilizer is diluted with from 3 to 8 times, preferably 5.5 times, its weight of distilled water before use.

By a cell culture medium is meant a nutrient medium which permits growth of cells in vitro. Some specific nutrient media are, for example, Medium 199, Morgan et al., Proc. Soc. Exp. Biol. & Med., 73:1—8, 1950; Basal Medium Eagle, Eagle, Science, 122, 501—504, 1955; In Vitro, Vol. 6, No. 2, 1970; Dulbecco's Modified Eagle's Medium, Dulbecco et al., Virology, 8, 396, 1959; Smith et al., J. Virol., 12, 185—196, 1960; In Vitro, Vol. 6, No. 2, 1970; Minimum Essential Medium (Eagle), Science, 130, 432 (1959) and RPMI Media, Moore et al., 199, 519—524, 1967; In Vitro, Vol. 6, No. 2, 1970.

The stabilizer of the present invention is applicable to various liquid virus vaccines such as, for example, measles, mumps, rubella, respiratory syncytial, parainfluenza types 1, 2 and 3, and cytomegalovirus.

The stabilizer composition of the present invention contains the following ingredients in the amounts indicated:

| Ingredient | Parts by weight/50 ml |
|---|---|
| Partially hydrolyzed gelatin | 1.5—2.1 |
| Monosaccharide or disaccharide | 7.0—13.0 |
| Nutrient medium (solids) | 0.4—0.6 |
| L-Glutamic acid | 0.35—0.7 |
| L-Arginine | 0.75—1.3 |
| Physiologically acceptable buffer to adjust pH to 6.0—6.5 | 0.05—0.2 M |

Specific preferred formulations of the liquid viral vaccine stabilizer of the present invention follow:

| | |
|---|---|
| Partially hydrolyzed gelatin | 1.8 g |
| Sucrose or Sorbitol | 10.0 g |
| Medium 199 | 0.55 g |

| Monosodium glutamate | 0.5 g |
| L-Arginine HCl | 1.0 g |
| Sodium phosphate buffer, 1M, pH 6.2 | 5 ml |
| Water, q.s. to | 50.0 ml |

In addition the stabilizer optionally but preferably contains a small amount of $NaHCO_3$ and of phenol red. In the case of the foregoing formulation the $NaHCO_3$ may be present in an amount of 1.2 g and the phenol red in an amount of 0.01 g. While particular formulations have been described above it is to be understood that variations in ratios and concentration of each ingredient are contemplated. One volume of bulk vaccine is usually diluted with from 2 to 12 volumes of stabilizer.

The following examples illustrate the present invention.

Example 1

40 Ml. of measles viral concentrate which has been stored at −70°C is thawed in a water bath at 25°C and then kept at 4—8°C. The liquid viral concentrate is then split into two aliquots each 16.5 ml.

A) one 16.5 ml aliquot from this virus fluid is diluted in 50 ml of the following stabilizer which has been sterilized by passing through a 0.2 $\mu$m membrane.

| 1M Phosphate buffer, pH 6.2 | 5.0 ml |
| Sorbitol, 25% aqueous solution | 7.1 ml |
| Hydrolyzed gelatin, 25% aqueous solution | 7.1 ml |
| Medium 199 | 30.7 ml |

Formulation is carried on under aseptic conditions and laminar flow hood. To prevent microbial growth, Neomycin (0.1 ml, 2500 units) is added to the preparation. The diluted vaccine is dispensed into 2 ml glass ampules (0.7 ml vaccine per ampule) which are immediately flame sealed and stored at 37°C.

B) The second 16.5 ml aliquot is handled as the first, except that the stabilizer formulation additionally contains 0.5 g monosodium glutamate 1.0 g L-arginine HCl and 10 g sucrose instead of sorbitol.

The storage stability of the vaccines is described in the following table:

Titers[1] of liquid vaccines stored at 37°C

| Time | Stabilizer of formulation A | Stabilizer of formulation B |
|---|---|---|
| 0 | 3.9 | 3.7 |
| 24 hours | 2.3 | 3.1 |
| 48 hours | 2.0 | 2.7 |
| 72 hours | 1.5 | 2.5 |

[1]Titers are expressed as $TCID_{50}/0.1$ ml.

Example 2

25 Ml of measles viral concentrate which has been stored at −70°C is thawed in a water bath at 25°C and then kept at 4—8°C. The liquid viral concentrate is diluted in 75 ml of the B stabilizer formulation (Example 1) and dispensed into 2 ml glass ampules. The ampules are heat sealed and stored at 2—8°C. The storage stability of the vaccine is described in the following table:

Stability of liquid vaccine stored at 2—8°C

| Time (months) | Titer ($TCID_{50}/0.1$ ml) |
|---|---|
| 0 | 3.7 |
| 1 | 4.1 |
| 2 | 3.1 |
| 4 | 3.5 |
| 6 | 3.3 |

Example 3

The following table compares the stability of four lots of liquid measles vaccines prepared at varying times using the stabilizers of formulation A or B as described in Example 1.

4

| | Lot | Stabilizer | Titer (TCID$_{50}$/0.1 ml) after hours indicated at 37°C | | | |
|---|---|---|---|---|---|---|
| | | | 0 | 24 | 48 | 72 |
| | 1 | A | 3.9 | 2.3 | 2.0 | 1.5 |
| | 1 | B | 3.7 | 3.1 | 2.7 | 2.5 |
| | 2 | A | 4.0 | 3.1 | 2.5 | 1.8 |
| | 2 | B | 3.9 | 3.6 | 3.0 | 2.6 |
| | 3 | A | 3.9 | — | 2.2 | 2.1 |
| | 3 | B | 3.6 | 3.3 | 3.0 | 2.5 |
| | 4 | A | 3.5 | 2.1 | 1.6 | 1.5 |
| | 4 | B | 3.6 | 3.5 | 3.1 | 2.4 |

**Claims for the contracting states: BE, CH, DE, FR, GB, IT, LI, LU, NL, SE**

1. A liquid vaccine comprising an inactivated or attenuated virus and a stabilizer consisting essentially of on a parts by weight basis from 1.5 to 2.1 parts partially hydrolyzed gelatin having a molecular weight of 3,000 from 7.0 to 13.0 parts of a monosaccharide, disaccharide or the polyhydric alcohol sorbitol from 0.4 to 0.6 part of an *in vitro* cell culture medium, and an amount of a physiologically acceptable acidic buffer effective to maintain the pH to from 6.0 to 6.5, characterized by the fact it also comprises from 0.35 to 0.7 part L-glutamic acid, from 0.75 to 1.3 parts L-arginine.

2. A vaccine according to claim 1, characterized by the fact that the disaccharide is sucrose.

3. A vaccine according to claim 1, characterized by the fact that the buffer is phosphate buffer.

4. A vaccine according to claim 1, characterized by the fact that the virus is measles, mumps, rubella, varicella, polio or hepatitis, herpes simplex type 1, herpes simplex type 2 or combinations thereof.

5. A vaccine according to claim 1, characterized by the fact that the stabilizer consists essentially of on a parts by weight basis of 1.8 parts partially hydrolyzed gelatin having a molecular weight of 3,000, 10.0 parts of a monosaccharide, disaccharide or polyhydric alcohol, 0.5 part of an *in vitro* cell culture medium, 0.5 part L-glutamic acid, 1.0 part L-arginine, and an amount of a physiologically acceptable acidic buffer effective to adjust the pH to from 6.0 to 6.5.

6. A vaccine according to claim 5, characterized by the fact that the disaccharide is sucrose.

7. A stabilizer for a liquid vaccine consisting essentially of on a parts by weight basis from 1.5 to 2.1 parts partially hydrolyzed gelatin having a molecular weight of 3,000, from 7.0 to 13.0 parts of a monosaccharide, disaccharide or the polyhydric alcohol sorbitol, from 0.4 to 0.6 part of an *in vitro* cell culture medium, and an amount of a physiologically acceptable acidic buffer effective to adjust the pH to from 6.0 to 6.5, characterized by the fact it also comprises from 0.35 to 0.7 part L-glutamic acid, from 0.75 to 1.3 parts L-arginine.

8. A stabilizer according to claim 7, characterized by the fact that the disaccharide is sucrose.

9. A stabilizer according to claim 7, characterized by the fact it consists essentially of on a parts by weight basis of 1.8 parts partially hydrolyzed gelatin having a molecular weight of 3,000, 10 parts of a monosaccharide, disaccharide or the polyhydric alcohol sorbitol, 0.5 part of an *in vitro* cell culture medium, 0.5 part L-glutamic acid, 1.0 part L-arginine, and an amount of a physiologically acceptable acidic buffer effective to adjust the pH to from 6.0 to 6.5.

10. A stabilizer according to claim 9, characterized by the fact that the disaccharide is sucrose.

**Claims for the contracting state: AT**

1. A process for preparing a liquid vaccine comprising mixing an inactivated or attenuated virus and a stabilizer consisting essentially of on a parts by weight basis from 1.5 to 2.1 parts partially hydrolyzed gelatin having a molecular weight of 3,000 from 7.0 to 13.0 parts of a monosaccharide, disaccharide or the polyhydric alcohol sorbitol from 0.4 to 0.6 part of an *in vitro* cell culture medium, and an amount of a physiologically acceptable acidic buffer effective to maintain the pH to from 6.0 to 6.5, characterized by the fact it also comprises from 0.35 to 0.7 part L-glutamic acid, from 0.75 to 1.3 parts L-arginine.

2. A process according to claim 1, characterized by the fact that the disaccharide is sucrose.

3. A process according to claim 1, characterized by the fact that the buffer is phosphate buffer.

4. A process according to claim 1, characterized by the fact that the virus is measles, mumps, rubella, varicella, polio or hepatitis, herpes simplex type 1, herpes simplex type 2 or combinations thereof.

5. A process according to claim 1, characterized by the fact that the stabilizer consists essentially of on a parts by weight basis of 1.8 parts partially hydrolyzed gelatin having a molecular weight of 3,000, 10.0 parts of a monosaccharide, disaccharide or polyhydric alcohol, 0.5 part of an *in vitro* cell culture medium, 0.5 part L-glutamic acid, 1.0 part L-arginine, and an amount of a physiologically acceptable acidic buffer effective to adjust the pH to from 6.0 to 6.5.

6. A process according to claim 5, characterized by the fact that the disaccharide is sucrose.

7. A process for preparation of a liquid vaccine by mixing essentially of on a parts by weight basis from 1.5 to 2.1 parts partially hydrolyzed gelatin having a molecular weight of 3,000, from 7.0 to 13.0 parts of a monosaccharide, disaccharide or the polyhydric alcohol sorbitol, from 0.4 to 0.6 part of an *in vitro* cell culture medium, and an amount of a physiologically acceptable acidic buffer effective to adjust the pH to from 6.0 to 6.5, characterized by the fact it also comprises from 0.35 to 0.7 part L-glutamic acid, from 0.75 to 1.3 parts L-arginine.

8. A process according to claim 7, characterized by the fact that the disaccharide is sucrose.

9. A process according to claim 7, characterized by the fact it consists essentially of on a parts by weight basis of 1.8 parts partially hydrolyzed gelatin having a molecular weight of 3,000, 10 parts of a monosaccharide, disaccharide or the polyhydric alcohol sorbitol, 0.5 part of an *in vitro* cell culture medium, 0.5 part L-glutamic acid, 1.0 part L-arginine, and an amount of a physiologically acceptable acidic buffer effective to adjust the pH to from 6.0 to 6.5.

10. A process according to claim 9, characterized by the fact that the disaccharide is sucrose.

**Patentansprüche für die Vertragsstaaten: BE, CH, DE, FR, GB, IT, LU, NL, SE**

1. Flüssige Vaccine, enthaltend ein inaktiviertes oder vermindertes Virus und einen Stabilisator, der im wesentlichen besteht auf der Basis von Gewichtsteilen aus 1,5 bis 2,1 Teilen teilweise hydrolysierter Gelatine mit einem Molekulargewicht von 3000, 7,0 bis 13,0 Teilen eines Monosaccharids, Disaccharids oder des mehrwertigen Alkohols Sorbit, 0,4 bis 0,6 Teilen eines in-vitro-Zellkulturmediums und einer Menge eines physiologisch brauchbaren sauren Puffers, die wirksam ist, um den pH-Wert bei 6,0 bis 6,5 zu halten, dadurch gekennzeichnet, daß sie auch 0,35 bis 0,7 Teile L-Glutaminsäure, 0,75 bis 1,3 Teile L-Arginin enthält.

2. Vaccine nach Anspruch 1, dadurch gekennzeichnet, daß das Disaccharid Saccharose ist.

3. Vaccine nach Anspruch 1, dadurch gekennzeichnet, daß der Puffer Phosphatpuffer ist.

4. Vaccine nach Anspruch 1, dadurch gekennzeichnet, daß das Virus Masern, Mumps, Röteln, Windpocken, Polio oder Hepatitis, Herpes simplex Typ I, Herpes simplex Typ II oder Kombinationen davon ist.

5. Vaccine nach Anspruch 1, dadurch gekennzeichnet, daß der Stabilisator im wesentlichen aus auf der Basis von Gewichtsteilen 1,8 Teilen teilweise hydrolysierter Gelatine mit einem Molekulargewicht von 3000, 10,0 Teilen eines Monosaccharids, oder mehrwertigen Alkohols, 0,5 Teilen eines in-vitro-Zellkulturmediums, 0,5 Teilen L-Glutaminsäure, 1,0 Teilen L-Arginin und einer Menge eines physiologisch brauchbaren sauren Puffers, die wirksam ist, den pH-Wert auf 6,0 bis 6,5 einzustellen, besteht.

6. Vaccine nach Anspruch 5, dadurch gekennzeichnet, daß das Disaccharid Saccharose ist.

7. Stabilisator für eine flüssige Vaccine, bestehend im wesentlichen aus auf Gewichtsbasis 1,5 bis 2,1 Teilen teilweise hydrolysierter Gelatine mit einem Molekulargewicht von 3000, 7,0 bis 13,0 Teilen eines Monosaccharids, Disaccharids oder des mehrwertigen Alkohols Sorbit, 0,4 bis 0,6 Teilen eines in-vitro-Zellkulturmediums und einer Menge eines physiologisch brauchbaren sauren Puffers, die wirksam ist, den pH-Wert auf 6,0 bis 6,5 einzustellen, dadurch gekennzeichnet, daß er auch 0,35 bis 0,7 Teile L-Glutaminsäure, 0,75 bis 1,3 Teile L-Arginin enthält.

8. Stabilisator nach Anspruch 7, dadurch gekennzeichnet, daß das Disaccharid Saccharose ist.

9. Stabilisator nach Anspruch 7, dadurch gekennzeichnet, daß er im wesentlichen besteht aus auf der Basis von Gewichtsteilen 1,8 Teilen teilweise hydrolysierter Gelatine mit einem Molekulargewicht von 3000, 10 Teilen eines Monosaccharids, Disaccharids oder des mehrwertigen Alkohols Sorbit, 0,5 Teilen eines in-vitro-Zellkulturmediums, 0,5 Teilen L-Glutaminsäure, 1,0 Teilen L-Arginin und einer Menge eines physiologisch brauchbaren sauren Puffers, die wirksam ist, den pH-Wert auf 6,0 bis 6,5 einzustellen.

10. Stabilisator nach Anspruch 9, dadurch gekennzeichnet, daß das Disaccharid Saccharose ist.

**Patentansprüche für den Vertragsstaat: AT**

1. Verfahren zur Herstellung einer flüssigen Vaccine durch Vermischen eines inaktivierten oder verminderten Virus und eines Stabilisators, der im wesentlichen besteht aus auf der Basis von Gewichtsteilen 1,5 bis 2,1 Teilen teilweise hydrolysierter Gelatine mit einem Molekulargewicht von 3000, 7,0 bis 13,0 Teilen eines Monosaccharids, Disaccharids oder des mehrwertigen Alkohols Sorbit, 0,4 bis 0,6 Teilen eines in-vitro-Zellkulturmediums und einer Menge eines physiologisch brauchbaren sauren Puffers, die wirksam ist, den pH-Wert bei 6,0 bis 6,5 zu halten, dadurch gekennzeichnet, daß sie auch 0,35 bis 0,7 Teile L-Glutaminsäure, 0,75 bis 1,3 Teile L-Arginin enthält.

2. Verfahren nach Anspruch 1, dadurch gekennzeichnet, daß das Disaccharid Saccharose ist.

3. Verfahren nach Anspruch 1, dadurch gekennzeichnet, daß der Puffer Phosphatpuffer ist.

4. Verfahren nach Anspruch 1, dadurch gekennzeichnet, daß das Virus Masern, Mumps, Röteln, Windpocken, Polio oder Hepatitis, Herpes simplex Typ I, Herpes simplex Typ II oder Kombinationen davon ist.

5. Verfahren nach Anspruch 1, dadurch gekennzeichnet, daß der Stabilisator im wesentlichen besteht aus auf der Basis von Gewichtsteilen 1,8 Teilen teilweise hydrolysierter Gelatine mit einem Molekulargewicht von 3000, 10,0 Teilen eines Monosaccharids, Disaccharids oder mehrwertigen Alkohols, 0,5 Teilen eines in-vitro-Zellkulturmediums, 0,5 Teilen L-Glutaminsäure, 1,0 Teilen L-Arginin und einer Menge eines physiologisch brauchbaren sauren Puffers, die wirksam ist, den pH-Wert auf 6,0 bis 6,5 einzustellen.

6. Verfahren nach Anspruch 5, dadurch gekennzeichnet, daß das Disaccharid Saccharose ist.

7. Verfahren zur Herstellung einer flüssigen Vaccine durch Vermischen von im wesentlichen auf der Basis von Gewichtsteilen 1,5 bis 2,1 Teilen teilweise hydrolysierter Gelatine mit einem Molekulargewicht von 3000, 7,0 bis 13,0 Teilen eines Monosaccharids, Disaccharids oder des mehrwertigen Alkohols Sorbit, 0,4 bis 0,6 Teilen eines in-vitro-Zellkulturmediums und einer Menge eines physiologisch brauchbaren sauren Puffers, die wirksam ist, den pH-Wert auf 6,0 bis 6,5 einzustellen, dadurch gekennzeichnet, daß sie auch 0,35 bis 0,7 Teile L-Glutaminsäure, 0,75 bis 1,3 Teile L-Arginin enthält.

8. Verfahren nach Anspruch 7, dadurch gekennzeichnet, daß das Disaccharid Saccharose ist.

9. Verfahren nach Anspruch 7, dadurch gekennzeichnet, daß es im wesentlichen besteht aus auf der Basis von Gewichtsteilen 1,8 Teilen teilweise hydrolysierter Gelatine mit einem Molekulargewicht von 3000, 10 Teilen eines Monosaccharids, Disaccharids oder des mehrwertigen Alkohols Sorbit, 0,5 Teilen eines in-vitro-Zellkulturmediums, 0,5 Teilen L-Glutaminsäure, 1,0 Teilen L-Arginin und einer Menge eines physiologisch brauchbaren sauren Puffers, die wirksam ist, den pH-Wert auf 6,0 bis 6,5 einzustellen.

10. Verfahren nach Anspruch 9, dadurch gekennzeichnet, daß das Disaccharid Saccharose ist.

**Revendications pour les etats contractants: BE, CH, DE, FR, GB, IT, LU, NL, SE**

1. Vaccin liquide comprenant un virus inactivé ou atténué et un stabilisant constitué essentiellement en parties en poids de 1,5 à 2,1 parties de gélatine partiellement hydrolysée ayant un poids moléculaire de 3,000, de 7,0 à 13,0 parties d'un monosaccharide, disaccharide ou du polyolsorbitol, de 0,4 à 0,6 partie d'un milieu de culture cellulaire in vitro et d'une quantité d'un tampon acide physiologiquement acceptable efficace pour maintenir le pH entre 6,0 et 6,5, caractérisé par le fait qu'il comprend également de 0,35 à 0,7 partie d'acide L-glutamique et de 0,75 à 1,3 partie de L-arginine.

2. Vaccin selon la revendication 1, caractérisé par le fait que le disaccharide est le saccharose.

3. Vaccin selon la revendication 1, caractérisé par le fait que le tampon est un tampon phosphate.

4. Vaccin selon la revendication 1, caractérisé par le fait que le virus est le virus morbilleux, le virus des oreillons, le virus de la rubéole, le virus de la varicelle, le virus de la poliomyélite ou de l'hépatite, le virus de l'herpes simplex type 1, le virus de l'herpes simplex type 2 ou une de leurs combinaisons.

5. Vaccin selon la revendication 1, caractérisé par le fait que le stabilisant est constitué essentiellement en parties en poids de 1,8 partie de gélatine partiellement hydrolysée ayant un poids moléculaire de 3,000, 10,0 parties d'un monosaccharide, disaccharide ou polyol, 0,5 partie d'un milieu de culture cellulaire in vitro, 0,5 partie d'acide L-glutamique, 1,0 partie de L-arginine et d'une quantité d'un tampon acide physiologiquement acceptable efficace pour ajuster le pH entre 6,0 et 6,5.

6. Vaccin selon la revendication 5, caractérisé par le fait que le disaccharide est le saccharose.

7. Stabilisant pour un vaccin liquide constitué essentiellement en parties en poids de 1,5 à 2,1 parties de gélatine partiellement hydrolysée ayant un poids moléculaire de 3,000, de 7,0 à 13,0 parties d'un monosaccharide, disaccharide ou du polyolsorbitol, de 0,4 à 0,6 partie d'un milieu de culture cellulaire in vitro et d'une quantité d'un tampon acide physiologiquement acceptable efficace pour ajuster le pH entre 6,0 et 6,5, caractérisé par le fait qu'il comprend également de 0,35 à 0,7 partie d'acide L-glutamique et de 0,75 à 1,3 partie de L-arginine.

8. Stabilisant selon la revendication 7, caractérisé par le fait que le disaccharide est le saccharose.

9. Stabilisant selon la revendication 7, caractérisé par le fait qu'il est constitué essentiellement en parties en poids de 1,8 partie de gélatine partiellement hydrolysée ayant un poids moléculaire de 3,000, 10 parties d'un monosaccharide, disaccharide ou du polyolsorbitol, 0,5 partie d'un milieu de culture cellulaire in vitro, 0,5 partie d'acide L-glutamique, 1,0 partie de L-arginine et d'une quantité d'un tampon acide physiologiquement acceptable efficace pour ajuster le pH entre 6,0 et 6,5.

10. Stabilisant selon la revendication 9, caractérisé par le fait que le disaccharide est le saccharose.

**Revendications pour l'etat contractant: AT**

1. Procédé pour la préparation d'un vaccin liquide comprenant le mélange d'un virus inactivé ou atténué et d'un stabilisant constitué essentiellement en parties en poids de 1,5 à 2,1 parties de gélatine partiellement hydrolysée ayant un poids moléculaire de 3,000, de 7,0 à 13,0 parties d'un monosaccharide, disaccharide ou du polyolsorbitol, de 0,4 à 0,6 partie d'un milieu de culture cellulaire in vitro et d'une quantité d'un tampon acide physiologiquement acceptable efficace pour maintenir le pH entre 6,0 et 6,5, caractérisé par le fait qu'il comprend aussi de 0,35 à 0,7 partie d'acide L-glutamique et de 0,75 à 1,3 partie de L-arginine.

2. Procédé selon la revendication 1, caractérisé par le fait que le disaccharide est le saccharose.

3. Procédé selon la revendication 1, caractérisé par le fait que le tampon est un tampon phosphate.

4. Procédé selon la revendication 1, caractérisé par le fait que le virus est le virus morbilleux, le virus des oreillons, le virus de la rubéole, le virus de la varicelle, le virus de la poliomyélite ou de l'hépatite, le virus de l'herpes simplex type 1, le virus de l'herpes simplex type 2 ou une de leurs combinaisons.

5. Procédé selon la revendication 1, caractérisé par le fait que le stabilisant est constitué essentiellement en parties en poids de 1,8 partie de gélatine partiellement hydrolysée ayant un poids moléculaire de 3,000, 10,0 parties d'un monosaccharide, disaccharide ou polyol, 0,5 partie d'un milieu de culture cellulaire in vitro, 0,5 partie d'acide L-glutamique, 1,0 partie de L-arginine et d'une quantité d'un tampon acide physiologiquement acceptable efficace pour ajuster le pH entre 6,0 et 6,5.

6. Procédé selon la revendication 5, caractérisé par le fait que le disaccharide est le saccharose.

7. Procédé pour la préparation d'un vaccin liquide par mélange essentiellement en parties en poids de 1,5 à 2,1 parties de gélatine partiellement hydrolysée ayant un poids moléculaire de 3,000, de 7,0 à 13,0 parties d'un monosaccharide disaccharide ou du polyolsorbitol, de 0,4 à 0,6 partie d'un milieu de culture cellulaire in vitro et d'une quantité d'un tampon acide physiologiquement acceptable efficace pour ajuster le pH entre 6,0 et 6,5, caractérisé par le fait qu'il comprend également de 0,35 à 0,7 partie d'acide L-glutamique et de 0,75 à 1,3 partie de L-arginine.

8. Procédé selon la revendication 7, caractérisé par le fait que le disaccharide est le saccharose.

9. Procédé selon la revendication 7, caractérisé par le fait qu'il est constitué essentiellement en partie en poids de 1,8 partie de gélatine partiellement hydrolysée ayant un poids moléculaire de 3,000, 10 parties d'un monosaccharide, disaccharide ou du polyolsorbitol, 0,5 partie d'un milieu de culture cellulaire in vitro, 0,5 partie d'acide L-glutamique, 1,0 partie de L-arginine et d'une quantité d'un tampon acide physiologiquement acceptable efficace pour ajuster le pH de 6,0 à 6,5.

10. Procédé selon la revendication 9, caractérisé par le fait que le disaccharide est le saccharose.